# EUROPEAN PATENT APPLICATION

(11) **EP 1 867 339 A1**
(43) Date of publication of application: **19.12.2007**
(21) Application number: 06715046.6
(22) Date of filing: 02.03.2006
(51) Int. Cl.: A61K 38/18, A61K 31/7056, A61K 38/21, A61K 45/00, A61K 45/06, A61P 31/14, A61P 43/00

(54) **ANTI-VIRAL AGENT**

(30) Priority: 02.03.2005 JP 2005057699
(71) Applicant: Mitsubishi Pharma Corporation, Chuo-ku, Tokyo 103-8405 (JP)
(72) Inventor: ISHII, Takehisa MITSUBISHI PHARMA CORPORATION, Chuo-ku Tokyo 1038405 (JP); ITAMI, Seima MITSUBISHI PHARMA CORPORATION, Chuo-ku Tokyo 1038405 (JP)
(74) Representative: Merkle, Gebhard
(86) International application number: PCT/JP2006/303937
(87) International publication number: WO 2006/093211

(57) **Abstract**

An antiviral agent consisting of hepatocyte growth factor (HGF) or an agonist of the HGF receptor, and a medicament for prophylactic and/or therapeutic treatment of a disease caused by hepatitis C virus comprising using said agent simultaneously or separately with other anti-viral agent.

## Description

### Technical Field

The present invention relates to an antiviral agent.

### Background Art

Hepatitis C virus (hereinafter sometimes abbreviated as "HCV") is the primary cause of hepatitis, cirrhosis and liver cancer in Japan. It is estimated that there are about 1.5 million patients with chronic hepatitis and about 300,000 patients with cirrhosis in Japan, and among them, patients with the diseases caused by HCV account for 70 to 80%. Since HCV is infectious via blood, blood for transfusion has been screened, thereby infections via blood transfusion have been almost completely eradicated in recent days. However, there exist about two million carriers even at present, mainly consisting of the patients infected due to medical practices before then, which is a hotbed of new patients with chronic hepatitis.

Infection with HCV is only established in humans and chimpanzees, and no suitable *in vitro* infection or replication system has been available, and for these reasons, development of medicaments for hepatitis C has not progressed. Although variety of pharmaceutical preparations have been used for therapeutic treatment, those effective for improvement of viremia have been so far limited to various interferon (hereinafter sometimes abbreviated as "IFN") preparations and ribavirin. However, their effects are not satisfactory. Ribavirin alone is ineffective against HCV, and only a combinational application of ribavirin and IFN has been found to be effective. IFN alone is effective for 30% of patients, and the combinational use of both agents is effective for as high as 50% of patients. In particular, the curative possibility is very low in patients with HCV-1b type (genotype), which widely spreads especially in Japan, or those with a heavy viral load. Under the circumstances, it has been desired to develop medicaments effective for chronic hepatitis C, cirrhosis and liver cancer.

In addition to the antiviral agents, medicaments referred to as liver protecting agents are sometimes administered to patients with general chronic hepatitis including hepatitis C for inhibiting progression of necrosis of hepatocytes. Examples of the liver protecting agents that have been launched in the market include Stronger Neo-minophagen C, Adelavin No. 9 and Tathion as injections, and Glycyron, Thiola, Urso, Proheparum and Shosaikoto as oral agents, and the like. Although these agents improve liver function test values (leaking enzymes and the like) via stabilization of hepatocyte membranes or the like as a symptomatic treatment, they have no effect of eliminating viruses as the cause of the disease. Therefore, absolutely no efficacy of improving viremia can be expected.

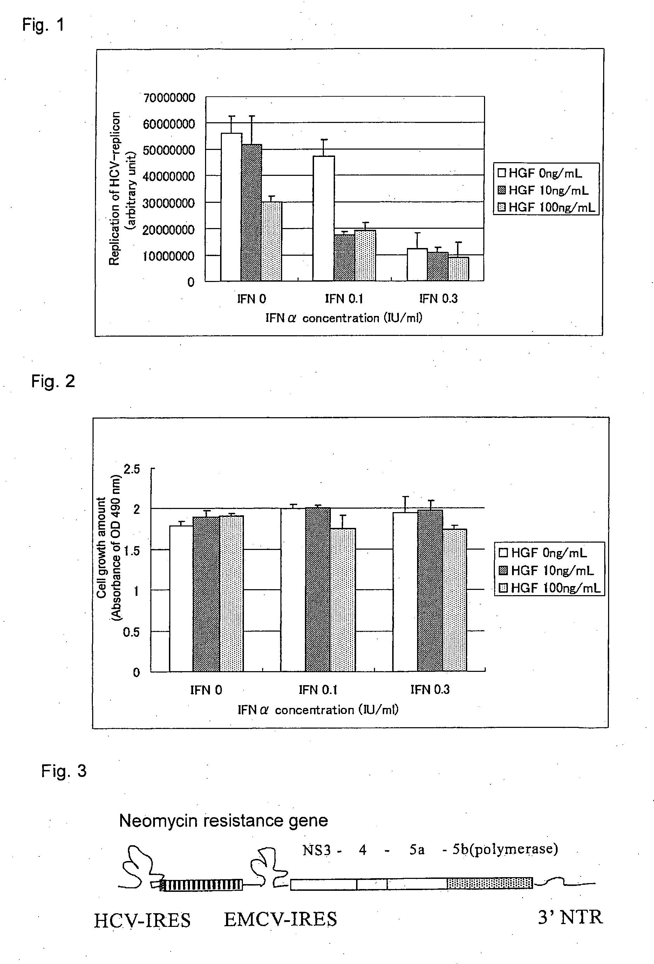

In recent years, an *in vitro* system that mimics the replication of hepatitis C virus called as HCV-subgenomic replicon has been established (Non-patent document 1). The HCV-subgenomic replicon is an HCV replication model intracellularly containing RNA (Fig. 3). An "HCV pseudo-RNA" having a neomycin resistance gene downstream from the HCV-internal ribosome entry site (IRES) and a HCV nonstructural gene sequence downstream from EMCV-IRES is transfected into the Huh7 cells, which are human liver cancer cell strain, and the transfected cells are selected in the presence of G418. In the resulting resistant clones, the replicon RNA autonomously replicates by HCV-derived polymerase, protease and the like and can serve as a model of HCV replication. This system is widely used in screening for anti-HCV agents. BILN-2061, an HCV protease inhibitor that inhibits replication of replicon RNA in this system was confirmed to improve viremia in patients with hepatitis C, which also revealed validity of this system (Non-patent document 2).

The hepatocyte growth factor (hereinafter also referred to as "HGF") was discovered as a potent growth factor of mature hepatocytes, and the gene thereof was cloned (Non-patent document 3). Subsequent studies have revealed that HGF is also involved in healing of wounds in the kidney, lung, stomach, duodenum, skin and the like in vivo, and that, as for the receptor of HGF, the c-Met proto-oncogene codes for the HGF receptor (Non-patent documents 4 and 5). At present, HGF is considered to be a factor that functions to repair tissues and regenerate organs via said receptor (Non-patent documents 6 and 7).

Especially in the field of liver diseases, both of applications for acute disease such as fulminant hepatitis, liver failure, and liver transplantation, and those for chronic disease such as chronic hepatitis and cirrhosis have been studied, in view of the hepatocyte growth promoting action, hepatocyte necrosis inhibiting action and liver function promoting action of HGF, and these medicinal applications are expected (Patent document 1). However, it has definitely never been verified yet whether or not HGF, per se, has a function of eliminating hepatitis virus directly.
Patent document 1: Japanese Patent Unexamined Publication (Kokai) No. 3-72883
Non-patent document 1: Science, 285, No. 5424, 110-113 (1999)
Non-patent document 2: Nature, 426, No. 6963, 186-189 (2003)
Non-patent document 3: Biochem. Biophys. Res. Commun., 163, 967 (1989)
Non-patent document 4: Science, 251, 802-804 (1991)
Non-patent document 5: Oncogene, 6, 501-504 (1991)
Non-patent document 6: Jikken Igaku (Experimental Medicine), 10, 144-153 (1992)
Non-patent document 7: Domyakukoka (Arteriosclerosis), 23, 683-688 (1996)

### Disclosure of the Invention

### Object to be Achieved by the Invention

An object of the present invention is to provide a novel antiviral agent.

### Means for Achieving the Object

The inventors of the present invention conducted various researches to achieve the foregoing object. As a result, they found that administration of HGF alone inhibited HCV replication, and that combinational use of HGF with an antiviral agent, including IFN as a typical example, enhanced the inhibition of HCV replication. The present invention was achieved on the basis of these findings.

Thus, the gists of the present invention are as follows.
(1) An antiviral agent consisting of hepatocyte growth factor (HGF) or an agonist of the HGF receptor.
(2) The antiviral agent according to (1), wherein the virus is hepatitis C virus.
(3) A pharmaceutical preparation characterized in using HGF or an agonist of the HGF receptor simultaneously or separately with one or more kinds of antiviral agents.
(4) The pharmaceutical preparation according to (3), wherein the antiviral agent is a substance having an inhibitory activity to the HCV-subgenomic replicon.
(5) The pharmaceutical preparation according to (3) or (4), wherein the antiviral agent consists of one or more kinds of substances selected from HCV protease inhibitors, HCV polymerase inhibitors and HCV helicase inhibitors.
(6) The pharmaceutical preparation according to (3), characterized in that the antiviral agent is an inosine monophosphate dehydrogenase (hereinafter also referred to as "IMPDH") inhibitor.
(7) The pharmaceutical preparation according to any one of (3) to (6), wherein the antiviral agent consists of one or two kinds of substances selected from interferons (IFN) and ribavirin.
(8) The pharmaceutical preparation according to any one of (3) to (7), which is used for prophylactic and/or therapeutic treatment of a disease caused by hepatitis C virus.
(9) The pharmaceutical preparation according to (8), wherein the disease caused by hepatitis C virus is selected from acute hepatitis, chronic hepatitis, cirrhosis and liver cancer.
(10) The pharmaceutical preparation according to any one of (3) to (9), which is for improvement of viremia.
(11) A method for prophylactic and/or therapeutic treatment of a disease caused by hepatitis C virus, which comprises using HGF or an agonist of the HGF receptor simultaneously or separately with one or more kinds of antiviral agents.
(12) The method for prophylactic and/or therapeutic treatment according to (11), wherein the antiviral agent is a substance having an inhibitory activity to the HCV-subgenomic replicon.
(13) The method for prophylactic and/or therapeutic treatment according to (11) or (12), wherein the antiviral agent consists of one or more kinds of substances selected from HCV protease inhibitors, HCV polymerase inhibitors and HCV helicase inhibitors.
(14) The method for prophylactic and/or therapeutic treatment according to (11), characterized in that the antiviral agent is an IMPDH inhibitor.
(15) The method for prophylactic and/or therapeutic treatment according to any one of (11) to (14), wherein the antiviral agent consists of one or two kinds of substances selected from IFNs and ribavirin.
(16) The method for prophylactic and/or therapeutic treatment according to any one of (11) to (15), wherein the disease caused by hepatitis C virus is selected from acute hepatitis, chronic hepatitis, cirrhosis and liver cancer.
(17) A method for improvement of viremia, which comprises using HGF or an agonist of the HGF receptor simultaneously or separately with one or more kinds of antiviral agents.
(18) The method for improvement according to (17), wherein the antiviral agent is a substance having an inhibitory activity to the HCV-subgenomic replicon.
(19) The method for improvement according to (17) or (18), wherein the antiviral agent consists of one or more kinds of substances selected from HCV protease inhibitors, HCV polymerase inhibitors and HCV helicase inhibitors.
(20) The method for improvement according to (17), characterized in that the antiviral agent is an IMPDH inhibitor.
(21) The method for improvement according to any one of (17) to (20), wherein the antiviral agent consists of one or two kinds of substances selected from IFNs and ribavirin.

### Effect of the Invention

According to the present invention, a novel antiviral agent can be provided.

### Brief Description of the Drawings

[Fig. 1] A graph showing inhibitory effects of IFN and/or HGF against HCV-replicon replication in HCV-replicon cells at various concentrations. The horizontal axis indicates IFN concentrations (IU/ml), and the vertical axis indicates amounts of the replicon replication (arbitrary unit in quantitative PCR). The amounts of added HGF at each IFN concentration are indicated by a graph of three bars in different colors. Further, the standard deviation for each sample is represented by a bar line.
[Fig. 2] A graph showing effects on cell growth action under the same experimental conditions as those in Fig. 1. The horizontal axis indicates IFN concentrations (IU/ml), and the vertical axis indicates the cell growth amounts (absorbance of OD490 nm). The amounts of added HGF at each IFN concentration are indicated as a graph of three bars in different colors. Further, the standard deviation for each sample is represented by a bar line.
[Fig. 3] A schematic view showing the structure of the HCV subgenomic replicon. Best Mode for Carrying out the Invention

Hereafter, the present invention will be explained in more detail.

HGF used in the present invention is a known substance described in Patent document 1 mentioned above.

It is known that HGF binds to the receptor thereof and thereby exhibits the actions (Non-patent document 4), and an effect similar to that obtained by using HGF according to the present invention can also be obtained with an agonist that acts on the HGF receptor. Therefore, according to the present invention, a similar effect can be expected when HGF derivatives, partial peptides, HGF-like low molecular weight compounds, anti-HGF receptor agonist antibodies and the like having an action similar to that of HGF are used instead of HGF. Examples of the HGF derivatives include the derivatives described in Nature, 342, 440-443 (1989) and the like. Examples of the anti-HGF receptor antibodies include the antibodies described in J. Cell Sci., 111, 237-247 (1998) and the like. Examples of the partial peptides include the HGF-derived partial peptides having an agonistic activity described in FEBS Lett., Vol. 22, 1-6, (1997) and the like. However, the substances used instead of HGF are not particularly limited to the aforementioned substances so far that the substances have an antagonistic effect on the HGF receptor. Further, known substances that do not directly act on the HGF receptor but act on HGF activators or HGF activator inhibitors, which are involved in the downstream signal transmission of the HGF receptor or HGF activation, may be included in the agonists of the HGF receptor according to the present invention.

The characteristic features of the present invention are based on the finding that the aforementioned HGF or an agonist of the HGF receptor can be used as an antiviral agent, and further based on the finding that a combinational use of HGF or an agonist of the HGF receptor with another antiviral agent enhances the inhibition of HCV replication by the antiviral agent.

The antiviral agents used in the present invention are not particularly limited so far that the substances have an inhibitory activity against the HCV-subgenomic replicon (Non-patent document 1). The substance that inhibits the HCV-subgenomic replicon herein referred to means an agent that is capable of reducing an amount of replicon RNA contained per one cell to 50% or less, more preferably to 10% or less, when the agent is added to the HCV-subgenomic replicon. Examples thereof include BILN2061 (Non-patent document 2) and the like, but not particularly limited so far that the agents have the aforementioned action. Specific examples include one or more kinds of substances selected from HCV protease inhibitors, HCV polymerase inhibitors and HCV helicase inhibitors.

Inosine monophosphate dehydrogenase (IMPDH) inhibitors, of which typical example includes ribavirin, are also known to have an anti-HCV activity when they are used in combination with an interferon (IFN) in humans. The IMPDH inhibitors are also included in the antiviral agents according to the present invention.

Specific examples include one or two kinds of substances selected from IFNs and ribavirin.

As the aforementioned HGF and IFN, those prepared by various methods can be used so far that they are purified to such an extent that they can be used as medicaments. For example, primary culture cells or established cell lines that produce HGF or IFN are cultured, and HGF or IFN can be obtained by isolation and purification from the culture supernatant or the like. Alternatively, the gene encoding HGF or IFN can be incorporated into an appropriate vector by a genetic engineering technique, and then the vector can be introduced into a suitable host cell for transformation and the target recombinant HGF or IFN can be obtained from the culture supernatant of the transformant. As the aforementioned host cell, various host cells conventionally used in genetic engineering techniques such as *Escherichia coli*, yeast, baculovirus (arthropod polyhedrosis virus)-insect cell or animal cell systems (see, Biochem. Biophys. Res. Commun., 175, 660 (1991) and Japanese Patent No. 2577091) and the like can be used, but the cells are not particularly limited to these examples.

The transformant introduced with the recombinant vector is cultured in a medium suitable for the transformant. The medium contains carbon source, nitrogen source, inorganic substances, vitamins, serum, which are required for growth of the transformant, agent used for screening based on resistance and the like. Specific examples include LB medium (Nacalai Tesque Inc.) and the like for the case where the host of the transformant is *Escherichia coli,* YPD medium (Genetic Engineering, vol. 1, p. 117, Plenum Press (1979)) and the like for the case where the host is yeast, Ham-12 medium, MEM medium, DMEM medium, RPMI1640 medium (SIGMA) containing 20% or less of fetal bovine serum, and the like for the case where the host is an insect cell or animal cell. Further, culture temperature, CO₂ concentration, and a period of time for culture can be suitably selected depending on the host, recombinant vector and the like. Further, aeration and agitation are performed, if necessary. Any medium composition or culture conditions other than those mentioned above can be used so far that the transformed host grows, and the protein encoded by the inserted HGF or IFN polynucleotide is produced.

As for a method for collecting the transformant cultured as described above, when the host is a cell, for example, a method of separating cells by centrifugation of the culture or the like, and then collecting the recombinant protein as cells or culture supernatant, or the like is used. Examples of the method for extracting the recombinant protein from the collected cells include ordinarily used known methods.

HGF or IFN can also be prepared by using a known cell-free protein synthesis system or the like. Specific examples of cell extracts used for the system include known cell extracts, for example, cell extracts of microorganisms such as *Escherichia coli*, germs of plant seeds, rabbit reticulocytes, and the like. Commercially available cell extracts can also be used, or cell extracts can also be prepared according to a method known per se, specifically, *Escherichia coli* extract can be prepared according to the method described in Pratt, J.M. et al., Transcription and Translation, Hames, B.D. & Higgins, S.J., p 179-209, IRL Press, Oxford (1984), or the like.

Further, as IFN, those marketed as reagents or medicaments can also be used. Examples thereof include IFN- α , IFN-β, IFN-γ, consensus IFN, and the like. Further, IFNs subjected to modification such as polyethylene glycolation can also be used. IFNs are not particularly limited to the aforementioned subtypes and modified IFNs so far that they are found to have antiviral effects.

As for HGF or IFN used in the present invention, one or more amino acids in the amino acid sequence thereof may be substituted, deleted and/or added so far that those modified substantially have the same action as that of the natural type substance, and in a similar manner, a sugar chain may be substituted, deleted and/or added.

Further, nucleic acids hybridizable with the nucleic acid shown in Patent document 1 in Northern blotting as an ordinary method and proteins derived therefrom may also be encompasses within the HGF referred to by the present invention. Specifically, Northern blotting can be performed by the method described in "Biotechnology Experiment Illustrated", vol. 2, Fundamentals of Genetic Analysis, Chapter 10, Northern hybridization (Shujunsha Co., Ltd.). However, the method can be performed according to generally used Northern blotting protocols which are not limited to the above method, in other words, the methods are not limited to the method described in the aforementioned reference and the method exemplified below.

For example, from a gel after agarose gel electrophoresis of RNA extracted from cells in a conventional manner, the RNA is transferred to a nitrocellulose or nylon membrane, and the resulting membrane is hybridized by using a probe derived from the nucleic acid mentioned in Patent document 1. Specifically, the membrane is incubated with a nucleic acid probe prepared by the nucleic acid mentioned in Patent document 1 in a hybridization buffer [5 x SSPE (750 mM NaCl, 43.3 mM NaPO₄ (pH 7.4), 6.25 mM EDTA), 50% formamide, 5 x Denhalt's solution (0.1% BSA, 0.1% Ficol 400, 0.1% PVP) and 0.5% SDS] at 42 to 65°C, thereby the probe and an objective RNA can be hybridized. Although the above example is given as a hybridization buffer, hybridization buffers are not particularly limited so far that they are for hybridization utilizable for ordinary Northern blotting. As the nucleic acid probe, probes labeled with radioactive isotopes (RI), those labeled with chemicals such as DIG, biotin and fluorescein, or those labeled with enzymes such as alkaline phosphatase and peroxidase in a conventional manner can be used. Detection of the hybridized nucleic acid probe can be performed by a method of direct exposure of an X-ray film for the RI labeling, a method of addition of an enzyme-labeled antibody against the chemical and successive incubation with a luminescent substance for exposure of an X-ray film for the chemical labeling, a method of incubation of the membrane with a luminescent substance for exposure of an X-ray film for the enzyme labeling, or the like. However, the methods are not particularly limited to the above methods so far that the hybridization of the nucleic acid probe and the RNA can be detected.

The hybridization can also be carried out with decreasing the salt concentration or increasing the formamide concentration in the hybridization buffer. Specifically, the hybridization can be carried out in a similar manner by using a buffer having an Na concentration in a range of from 150 to 800 mM. Further, the hybridization can also be carried out at a formamide concentration in a range of from 50 to 70%. Nucleic acids hybridized within these ranges of salt and formamide concentration at 42 to 65°C and proteins derived therefrom fall within the scope of the claims of the present application.

As ribavirin, those launched in the mark as pharmaceutical products can be used. Specifically, ribavirin marketed by Schering-Plough Corporation as Rebetol (registered trade name) can be used. As for a dose, the agent can be administered at a daily dose of 50 to 5000 mg, and is preferably orally taken twice a day at a daily dose of 600 to 800 mg. However, doses are not particularly limited to the aforementioned doses so far that the antiviral activity can be observed. Ribavirin marketed as a reagent by Merck (Product No. 555580) can also be used.

According to the present invention, a method for prophylactic and/or therapeutic treatment of a disease caused by hepatitis C virus can be provided, which is characterized to comprise using HGF or an agonist of the HGF receptor simultaneously or separately with an antiviral agent. Examples of the disease caused by hepatitis C virus referred to herein include acute hepatitis, chronic hepatitis, cirrhosis and liver cancer. Further, the present invention also provides a method for improvement of viremia by using HGF or an agonist of the HGF receptor in combination with an antiviral agent.

According to the present invention, HGF or an agonist of the HGF receptor can be administered solely or together with other antiviral agent, of which typical example includes IFN, or can be prepared as a pharmaceutical composition in the form of a pharmaceutical preparation together with suitable pharmaceutical additives, and administered. Dosage forms of such pharmaceutical composition are not particularly limited so far that they are ordinarily used, and ampoules for injection, lyophilized powders for injection and the like can be used. Manufactures of various dosage forms can be performed according to conventional manners by using well-known pharmaceutical additives available to those skilled in the art such as diluents and additives.

For example, lyophilized powders for injection can be prepared in a conventional manner by dissolving an effective amount of purified HGF and/or other antiviral agents mentioned above in a solvent for dilution and adding excipients, stabilizers, preservatives, soothing agents, pH modifiers and the like, if necessary. Further, ampoules of injection can be prepared by dissolving an effective amount of HGF or other antiviral agents mentioned above in a solvent for dilution, adding additives such as dissolving aids, buffers, isotonic agents, stabilizers, preservatives, soothing agents and pH modifiers, if necessary, and sterilizing the solution by ordinary heat sterilization, aseptic filtration or the like.

In the present invention, the terminology "to use HGF or an agonist of the HGF receptor simultaneously with another antiviral agent" should be construed to encompass both meanings of a combinational use thereof and use of a mixture thereof.

According to the present invention, in addition to the aforementioned parenteral administration, the medicament can be used as pharmaceutical preparations formulated in dosage forms suitable for oral administration, inhalation or external use in the form of solids preparations such as tablets, granules, capsules and powders or liquids such as solutions, suspensions, syrups, emulsions and lemonades prepared by using pharmaceutically acceptable carriers and the like. If necessary, auxiliary agents, stabilizers, wetting agent and other commonly used additives may be mixed in the aforementioned preparations.

Doses of the aforementioned various pharmaceutical preparations vary depending on the route of administration, the type of a disease, symptoms, body weight or age and the like of a patient, as well as the type of the medicament to be used and the like. In general, as for HGF, the administration can be conducted at about 1 to 200 mg or more per day for a single patient. It is preferable to apply for prophylactic and/or therapeutic treatment of hepatopathy by adjusting an average single dose of HGF contained as an active ingredient to about 5 to 100 mg. As for IFN, the agent has already been marketed with the indication of "improvement of viremia in chronic active hepatitis C". For example, Canferon-A (genetic recombinant IFN α-2a preparation, Takeda Pharmaceutical Co., Ltd.) is administered at a dose of 3,000,000 to 9,000,000 IU per day.

Further, according to the present invention, the HGF gene and/or the IFN gene can be used as an agent for gene therapy for prophylactic and/or therapeutic treatment of hepatopathy caused by hepatitis C virus, or an agent for cell therapy, in which the HGF gene and/or IFN gene is introduced into suitable cells, and the resulting cells are transplanted into a tissue. For example, the HGF gene and/or the IFN gene of the present invention is mixed with a lipofection reagent and the the mixture is administered to a living body, thereby local HGF and/or IFN can be maintained at a concentration required for prophylactic and/or therapeutic treatment of hepatopathy caused by hepatitis C virus. The administration of HGF and/or IFN according to the present invention can be performed with an appropriate dose, administration method and frequency over a period until effectiveness of the prophylactic and/or therapeutic treatment comes to be observed, or until reduction of the pathological conditions is achieved, although the administration depends on severity of a disease or response of a living body.

### Examples

The present invention will be explained more specifically with reference to the following examples. However, the present invention is not limited to the examples.

### (Example 1) Effects of HGF on inhibition of HCV-replicon replication by IFN in HCV-replicon cells

### (Materials and methods for experiment)

As a cell system for *in vitro* replication of HCV, an HCV-replicon cell clone #5-15 derived from the human liver cancer cell strain Huh7 was used (purchased from ReBLikon GmbH). The clone #5-15 was suspended in the Dulbecco's Eagle's MEM medium containing 2% fetal bovine serum, and seeded on a 96-well plate at 1.5 x 10⁴ cells/100 µl/well. As blank, wells added with the medium alone was prepared without seeding the cells (Day 0). After culture overnight in a humid incubator at 37°C under 5% CO₂ gas, 50 µl each of human recombinant IFN α (BIOMEDICAL LABORATORIES, Cat. No. 11105-1, lot No. #2122) and/or human recombinant HGF (produced in-house, Lot No. 920629) was added to each well. The final concentrations of IFNα were 0, 0.1 and 0.3 international unit (IU)/ml, the final concentrations of HGF were 0, 10 and 100 ng/ml, and the final volume was adjusted to 200 µl for all the wells. Three wells (triplicate) were prepared for each sample (Day 1). After culture for 48 hours, total RNA was extracted from each well by using ABI-PRIZM 6100 Nucleic Acid PrepStation (Applied Biosystems). Extraction was performed according to the operation manual of the apparatus. The solution of the extracted RNA was stored at -80°C (Day 3). The samples were thawed on the next day, and amounts of the HCV-replicon RNA were quantified by quantitative polymerase chain reaction (PCR). The quantification was performed according to the method of Takeuchi et al. (Takeuchi, T. et al., Gastroenterology, Vol. 116, 636-642 (1999)) by using ABI PRISM 7900 (Applied Biosystems).

RNAs used in the quantitative PCR for a standard curve were prepared by *in vitro* transcription using a vector obtained by ligating the gene of the HCV MKC-1A strain (Genbank accession No. D45172) to the downstream of the T7 promoter using T7 RiboMAX Express Large Scale RNA Production System (Promega), and it was confirmed that linearity was obtainable between 10² and 10⁸ in the aforementioned quantitative PCR.

### (Results)

When amounts in the HCV-replicon RNA were quantified by the aforementioned method for various combinations of IFN and HGF concentrations, the followings were revealed.
(1) At a high HGF concentration (100 ng/ml), HGF alone inhibited the replication of the HCV-replicon.
(2) At an IFN concentration sufficient to inhibit the replication of the HCV-replicon (0.3 IU/ml), effect of HGF was not observed.
(3) At an IFN concentration at which IFN partially acted on the inhibition of HCV-replicon replication (0.1 IU/ml), HGF synergistically acted to inhibit the replication of HCV-replicons (Fig. 1).

### (Example 2) Effects of IFN, HGF and combinational use thereof on the growth of HCV-replicon cell strain

### (Materials and method for experiment)

To determine whether or not the decreases in the replication of HCV-replicons observed in Example 1 were attributable to the decrease in the cell count itself, the following experiment was performed.
The clone #5-15 was suspended in Dulbecco's Eagle's MEM medium containing 2% fetal bovine serum, and seeded on a 96-well plate at 1.5 x 10⁴ cells/100 µl/well. As blank, wells added with a medium alone was prepared without seeding cells (Day 0). After culture overnight in a humid incubator at 37°C under 5% CO₂ gas, 50 µl each of human recombinant IFN α (BIOMEDICAL LABORATORIES, Cat. No. 11105-1, lot No. #2122) and/or human recombinant HGF (produced in-house, Lot No. 920629) was added to each well. The final concentrations of IFN α were 0, 0.1 and 0.3 international unit (IU)/ml, the final concentrations of HGF were 0, 10 and 100 ng/ml, and the final volume was adjusted to 200 µl for all the wells. Three wells (triplicate) were prepared for each sample (Day 1). After culture for 48 hours, the number of cells was measured by Cell Titer 96 Aqueous One Solution Cell Proliferation Assay (Promega, Lot #171755), which is a type of cell growth assay kit. The measurement was performed according to the manual of the assay kit. Specifically, 100 µ1 of culture supernatant was removed from each well, and 20 µl of One Solution Reagent included in the assay kit was added to each well. Then, the plate was incubated for one hour in a humid incubator containing 5% CO₂ set at 37°C, and then measured at absorbance 490 nm by using a 96-well plate reader.

### (Results)

At any concentrations of HGF and IFN α and any combinations thereof used in the experiment, it was not observed to inhibit the growth of the HCV-replicon cell line (Fig. 2).

### (Conclusion)

When the concentration of HGF was relatively high (100 ng/ml), it was observed that HGF alone had an inhibitory action on the replication of the HCV-replicon. The growth of the cells per se was not inhibited at all in this experiment, and accordingly, this inhibitory action on the virus replication is not attributable to the inhibition on the growth of the host cells. Since HGF possesses a hepatocyte growth-stimulating activity, the indications as an agent of improving a chronic liver disease or a liver protecting agent has conventionally been expected. The results, which the inventors first showed in the present invention, provide a novel use of HGF as an agent for prophylactic and/or therapeutic treatment of a disease caused by HCV.

Further, at the concentration at which IFN partially acted (0.1 IU/ml), HGF synergistically acted to inhibit the replication of the HCV-replicon. The growth of the cells per se was not inhibited at all in this experiment, and accordingly, this inhibitory action on the virus replication is not attributable to the inhibition on the growth of the host cells. Further, the IFN-dependent inhibition of virus replication was also synergistically enhanced by HGF (10 ng/ml) at which almost no inhibition of the replication was observed with HGF alone, suggesting that IFN and HGF inhibited the replication of the virus by different mechanisms. Conventional antiviral agents against HCV, including IFN, do not have sufficient potential to eliminate virus even when they are clinically administered at an extremely high dose. The results showed by the inventors suggest that HGF may enhance improvement of HCV viremia by a mechanism different from that of a conventional antiviral agent, and provide a promising and novel therapy for diseases caused by HCV based on a combinational use of HGF with an existing antiviral agent or use of a mixture thereof.

Further, various adverse effects of IFN have been reported. The adverse effects range widely from influenza-like symptoms, exanthema, leukopenia/thrombocytopenia and proteinuria, which are frequently observed immediately after the start of administration, to alopecia, ocular fundus hemorrhage, psychoneurosis, interstitial pneumonia, onset/aggravation of autoimmune diseases such as thyroid gland disease and aggravation of diabetes, which are observed after certain period of time from the administration. They include severe side effects, which often lead to the case to threaten patients' life or the case to discontinue the administration inevitably. The combinational use of HGF with IFN or a mixture preparation thereof, which was first provided by the inventors, can be expected to have medicinal effect equal to or higher than that conventionally achieved even at a lower IFN dose than the conventional dose, and therefore, has an effect of reducing these adverse effects caused by IFN. Industrial Applicability

Although the present invention are explained in detail by referring to specific embodiments thereof, it is apparent to those skilled in the art that various alterations and modifications are possible without departing from the spirit and scope of the present invention. According to the present invention, a novel antiviral agent can be provided.
This application is based on Japanese patent application filed on March 2, 2005 (Japanese Patent Application No. 2005-057699), and the entire disclosures thereof are incorporated herein by reference.

## Claims

1. An antiviral agent consisting of hepatocyte growth factor or an agonist of the hepatocyte growth factor receptor.

2. The antiviral agent according to claim 1, wherein the virus is hepatitis C virus.

3. A pharmaceutical preparation **characterized in** using hepatocyte growth factor or an agonist of the hepatocyte growth factor receptor simultaneously or separately with one or more kinds of antiviral agents.

4. The pharmaceutical preparation according to claim 3, wherein the antiviral agent is a substance having an inhibitory activity to the hepatitis C virus-subgenomic replicon.

5. The pharmaceutical preparation according to claim 3 or 4, wherein the antiviral agent consists of one or more kinds of substances selected from hepatitis C virus protease inhibitors, hepatitis C virus polymerase inhibitors and hepatitis C virus helicase inhibitors.

6. The pharmaceutical preparation according to claim 3, **characterized in that** the antiviral agent is an inosine monophosphate dehydrogenase inhibitor.

7. The pharmaceutical preparation according to any one of claims 3 to 6, wherein the antiviral agent consists of one or two kinds of substances selected from interferons and ribavirin.

8. The pharmaceutical preparation according to any one of claims 3 to 7, which is used for prophylactic and/or therapeutic treatment of a disease caused by hepatitis C virus.

9. The pharmaceutical preparation according to claim 8, wherein the disease caused by hepatitis C virus is selected from acute hepatitis, chronic hepatitis, cirrhosis and liver cancer.

10. The pharmaceutical preparation according to any one of claims 3 to 9, which is for improvement of viremia.

11. A method for prophylactic and/or therapeutic treatment of a disease caused by hepatitis C virus, which is **characterized in** using hepatocyte growth factor or an agonist of the hepatocyte growth factor receptor simultaneously or separately with and one or more kinds of antiviral agents.

12. The method for prophylactic and/or therapeutic treatment according to claim 11, wherein the antiviral agent is a substance having an inhibitory activity to the hepatitis C virus-subgenomic replicon.

13. The method for prophylactic and/or therapeutic treatment according to claim 11 or 12, wherein the antiviral agent consists of one or more kinds of substances selected from hepatitis C virus protease inhibitors, hepatitis C virus polymerase inhibitors and hepatitis C virus helicase inhibitors.

14. The method for prophylactic and/or therapeutic treatment according to claim 11, **characterized in that** the antiviral agent is an inosine monophosphate dehydrogenase inhibitor.

15. The method for prophylactic and/or therapeutic treatment according to any one of claims 11 to 14, wherein the antiviral agent consists of one or two kinds of substances selected from interferons and ribavirin.

16. The method for prophylactic and/or therapeutic treatment according to any one of claims 11 to 15, wherein the disease caused by hepatitis C virus is selected from acute hepatitis, chronic hepatitis, cirrhosis and liver cancer.

17. A method for improvement of viremia, which is **characterized in** using hepatocyte growth factor or an agonist of the hepatocyte growth factor receptor simultaneously or separately with one or more kinds of antiviral agents.

18. The improving method according to claim 17, wherein the antiviral agent is a substance having an inhibitory activity to the hepatitis C virus-subgenomic replicon.

19. The method for improvement according to claim 17 or 18, wherein the antiviral agent consists of one or more kinds of substances selected from hepatitis C virus protease inhibitors, hepatitis C virus polymerase inhibitors and hepatitis C virus helicase inhibitors.

20. The method for improvement according to claim 17, **characterized in that** the antiviral agent is an inosine monophosphate dehydrogenase inhibitor.

21. The method for improvement according to any one of claims 17 to 20, wherein the antiviral agent consists of one or two kinds of substances selected from interferons and ribavirin.
